(19) Europäisches Patentamt
European Patent Office
Office européen des brevets

(11) **EP 3 212 187 B1**

(12) **EUROPEAN PATENT SPECIFICATION**

(45) Date of publication and mention
of the grant of the patent:
**09.12.2020 Bulletin 2020/50**

(21) Application number: **15794485.1**

(22) Date of filing: **26.10.2015**

(51) Int Cl.:
*A61K 31/4375* (2006.01)    *A61K 31/05* (2006.01)
*A61K 31/192* (2006.01)    *A61K 31/352* (2006.01)
*A61K 31/366* (2006.01)    *A61P 3/00* (2006.01)
*A61P 9/00* (2006.01)

(86) International application number:
**PCT/EP2015/074762**

(87) International publication number:
**WO 2016/066588 (06.05.2016 Gazette 2016/18)**

(54) **SYNERGISTIC COMBINATIONS COMPRISING BERBERINE AND TYROSOL OR TYROSOL AND FERULIC ACID WHICH STIMULATE THE EXPRESSION OF SIRTUIN 1**

SYNERGISTISCHE KOMBINATIONEN AUS BERBERIN UND TYROSOL ODER TYROSOL UND FERULINSÄURE ZUR STIMULIERUNG DER EXPRESSION VON SIRTUIN 1

COMBINAISONS SYNERGIQUES COMPRENANT DE LA BERBERINE ET DU TYROSOL OU DU TYROSOL ET DE L'ACIDE FERULIQUE STIMULANT L'EXPRESSION DE SIRTUINE 1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2014 IT MI20141843**

(43) Date of publication of application:
**06.09.2017 Bulletin 2017/36**

(73) Proprietors:
• **Giovannini, Luca**
  **I-56100 Pisa (IT)**
• **Laboratori Aliveda SRL Unipersonale**
  **56042 Crespina (PI) (IT)**

(72) Inventor: **GIOVANNINI Luca**
**56100 Pisa (IT)**

(74) Representative: **Bianchetti Bracco Minoja S.r.l.**
**Via Plinio, 63**
**20129 Milano (IT)**

(56) References cited:
**WO-A1-2008/156627      WO-A1-2012/106761**
**US-A1- 2007 116 779      US-A1- 2011 229 535**
**US-A1- 2013 017 283**

• **HOWITZ K T ET AL: "SMALL MOLECULE ACTIVATORS OF SIRTUINS EXTEND SACCHAROMYCES CEREVISIAE LIFESPAN", NATURE, NATURE PUBLISHING GROUP, UNITED KINGDOM, vol. 425, 11 September 2003 (2003-09-11), pages 191-196, XP001188967, ISSN: 0028-0836, DOI: 10.1038/NATURE01960**
• **DAVID J BONDA ET AL: "The sirtuin pathway in ageing and Alzheimer disease: mechanistic and therapeutic considerations", LANCET NEUROLOGY, vol. 10, no. 3, 1 March 2011 (2011-03-01), pages 275-279, XP055239782, GB ISSN: 1474-4422, DOI: 10.1016/S1474-4422(11)70013-8**
• **Samson Mathews Samuel ET AL: "Akt/FOXO3a/SIRT1-Mediated Cardioprotection by n -Tyrosol against Ischemic Stress in Rat in Vivo Model of Myocardial Infarction: Switching Gears toward Survival and Longevity", Journal of Agricultural and Food Chemistry, vol. 56, no. 20, 22 October 2008 (2008-10-22), pages 9692-9698, XP55546060, US ISSN: 0021-8561, DOI: 10.1021/jf802050h**

- **ANA P GOMES ET AL: "Berberine protects against high fat diet-induced dysfunction in muscle mitochondria by inducing SIRT1-dependent mitochondrial biogenesis", BIOCHIMICA ET BIOPHYSICA ACTA. MOLECULAR BASIS OF DISEASE, AMSTERDAM, NL, vol. 1822, no. 2, 7 October 2011 (2011-10-07), pages 185-195, XP028355418, ISSN: 0925-4439, DOI: 10.1016/J.BBADIS.2011.10.008 [retrieved on 2011-10-17]**
- **ALVALA RAVI ET AL: "Scientific evidence for traditional claim of anti-obesity activity ofTecomella undulatabark", JOURNAL OF ETHNOPHARMACOLOGY, ELSEVIER IRELAND LTD, IE, vol. 148, no. 2, 28 April 2013 (2013-04-28), pages 441-448, XP028571856, ISSN: 0378-8741, DOI: 10.1016/J.JEP.2013.04.033**

Remarks:
The file contains technical information submitted after the application was filed and not included in this specification

## Description

The invention relates to synergistic compositions comprising active ingredients capable of stimulating the expression of Sirtuin 1.

## State of the art

Sirtuins (SIRTs) have received great attention since their discovery, when it was attributed to them an important biochemical and molecular role in Saccharomyces cerevisiae, from which the name Sir2 (Silent Information Regulator 2) has been derived. Sir2, described as a regulator of gene silencing, can repress the expression of certain genes by deacetylation, also involved in the extension of lifespan in yeast.

Sir2 is able to repress the expression of certain genes by deacetylation of the $\varepsilon$-amino group of the N-terminal lysine residues of histones. The presence of four additional genes, homologous to Sir2 called HST 1-4 (homologues of Sir2), was identified in S. cerevisiae. The discovery of Sir2 homologues in yeast and shortly thereafter in bacteria, plants and mammals, demonstrated Sir2 is a member of a large, ancient family of genes now identified with the term "Sirtuins".

Mammals are known to possess seven different sirtuins (SIRT 1-SIRT 7), with varying subcellular localizations and chemical activities; the majority of the studies have focused on SIRT 1, the homologous gene of Sir2.

The role that these proteins can play in various diseases, especially for SIRT 1, is very important, mostly for its key functions in the body, both from a physiological and pathophysiological point of view. Indeed, SIRT 1 has a fundamental role in the regulation of many aspects of cellular processes ranging from energy metabolism to cell survival. It is involved in the regulation of chromatin genic transcription and in the modulation of several transcription factors such as p53, NF-kB, POC-1$\alpha$, FOXO, Ku70, PPAR and HSF-1, thereby interfering with the activity of genes related to growth, cell cycle, differentiation, cellular senescence, cellular apoptosis/proliferation, macroautophagy, energy metabolism and circadian transcription of several core clock genes, making a possible connection between lifespan and circadian rhythm.

In addition, this protein acts as a metabolic sensor and a mediator of survival under stress conditions, such as caloric restriction and exercise, conditions in which the SIRT 1 transcription is precisely activated.

SIRT 1 is able to increase the stability of telomerase, delaying the progressive telomere shortening and the input of the cells in replicative senescence and preserving the genomic integrity.

Given that the enzymatic activity of SIRT 1 depends on the presence of NAD +, it is clear that the activity of this protein is directly related to the regulation of lipid and carbohydrate metabolism.

As for lipid metabolism, it is able to inhibit lipogenesis and cholesterologenesis and to increase the fatty acids oxidation, lipolysis and mitochondrial efficiency.

On the other hand, as for glucose metabolism, it stimulates gluconeogenesis, inhibits glycolysis and acts as a sensor of insulin levels, by modulating the production of glucose-sensitive ATP and the resulting secretion of insulin from pancreatic beta-cells.

In addition, many recent studies have highlighted the important role of SIRT 1 also in the bile acids metabolism.

SIRT 1 is widely expressed in endothelial cells, where it controls important functions to suppress the development of atherosclerosis, including the upregulation of endothelial nitric oxide synthase (eNOS), the reduction of replicative senescence of vascular smooth muscle cells, the suppression of inflammation and ROS in the arteries and increase in vascular growth.

SIRT 1 acts as a cardioprotective molecule that protects the heart from aging, damage of ischemia/reperfusion, oxidative stress and hypertrophy, inhibits cardiomyocyte apoptosis and regulates energy metabolism rate.

In addition to this, it is able to improve the endothelium functions by reducing macrophage foam cells formation, and preventing calcification of vascular smooth muscle cells. Moreover, it is a key factor in the vascular development of mammals, because it modulates vascular growth, controls endothelium angiogenetic activity, regulates endothelial progenitor cell growth, and maintains endothelium homeostasis.

SIRT 1 inhibits oxidative stress, inflammation and promotes autophagy by interacting with several proteins, including FOXOs, NF-kB and mTOR, and with different signaling pathways.

Aging is recognized as the main risk factor for neurodegenerative diseases and, although this has been known already for many years, until the discovery of Sirtuins, and above all, of the important role of SIRT 1 in this context, it was not possible to use this pathway for the therapeutic treatment of neurodegeneration.

In Alzheimer's disease, SIRT 1 protects against A$\beta$-peptide neurotoxicity, through the inhibition of NF-kB signaling in microglia, and can directly deacetylate tau protein at multiple residues. This process decreases the accumulation of hyperphosphorylated tau protein, the cognitive defects and the premature mortality in P301L murine models for tauopathy.

On the other hand, regarding Parkinson's disease, SIRT 1 is able to decrease the formation of abnormal protein aggregates, whereas in Huntington's Chorea it plays a protective role against the neurotoxicity of the mutated protein. In fact, when is active, SIRT 1 can deacetylate TORC1, facilitating its interaction with CREB and the transcription of BDNF, a neurotrophic factor protective in Huntington's disease.

On the other hand, recently, many studies have shown an important link between SIRT 1 and SLA pathogenesis, noting that SIRT 1, by deacetylation of HSF1, might induce the transcription of molecular chaperones and thus decrease the neuronal death. In addition, other studies are instead focused on the relationship between SIRT 1 and cancer. SIRT 1 is an important molecule in this process, in fact it may improve genetic stability, increase epigenetic silencing, stimulate repair of DNA damages and lead to tumor suppression. In particular, SIRT 1 is able to interact with NF-kB, a factor that promotes the formation of metastases, Survivin, an anti-apoptotic gene, β-catenin and p53.

The p53 protein is involved in cell cycle control and referred to as "guardian of the genome", precisely for its role in preserving genomic stability by preventing mutations. However, when p53 is acetylated, it can activate the transcriptional program resulting in increased cell proliferation and apoptosis. Moreover, this transcription factor is hyperacetylated in cancer cells. The overexpression of SIRT 1, both in vitro and in vivo, in animal models, slows down cell proliferation and inhibits apoptosis, exactly through deacetylation of p53, indicating SIRT 1 as a potential target for anti-cancer drugs.

Besides all that, SIRT 1 is also located in the nucleus and in the cytoplasm of cells from all normal ocular structures, including the cornea, lens, iris, ciliary body and retina and plays a key role in eye diseases.

First of all, this protein is able to rescue neurons from Wallerian degeneration, and appears to have a neuroprotective effect in retinal damage, where it protects the retinal epithelial cells from DNA damage such as oxidative stress-related retinal damage, apoptotic retinal death and anti- inflammation. Furthermore, SIRT 1 is involved in the retinal vascular regeneration in ischemic proliferative retinopathy and is also able to mitigate UVB- related retinal damage, preventing phosphorylation of AKT and ERK.

Therefore, due to the key role of SIRT 1 in the body, compounds interacting with SIRT 1 pathway are able to slow the aging process, extend the lifespan and they can find pharmacological application in many disorders such as type 2 diabetes, acidosis, hyperglycemia, insulin resistance, metabolic syndrome, obesity, liver syndrome and steatosis, fatty liver, hypertriglyceridemia, hypercholesterolemia, hypertension, atherosclerosis, coronary artery disease, peripheral vascular disease, cardiac dysfunction, fibrosis, cardiac and ventricular hypertrophy, cardiac ischemia, hypoxia, asthma, rheumatoid arthritis, Parkinson's disease, Alzheimer's disease, Huntington's Chorea, amyotrophic lateral sclerosis, dementia with Lewy bodies, prion disease, frontotemporal dementia, Wallerian degeneration, macular degeneration, glaucoma, optic neuritis, retinal damage, ischemic proliferative retinopathy, diabetic retinopathy, retinitis pigmentosa, senile cataracts, keratitis, and also in all the carcinogenic degenerations of invasive ductal, invasive lobular, medullary, colloid, papillary and tubular type.

On the basis of what has been described, it is evident how substances or groups of substances, able to increase SIRT 1 expression, may find a pharmacological use, taking also into account thatSIRT 1 over-expression proved non to involve toxic activity.

This is justified also by many studies in the literature on animal models, which demonstrate how the use of other SIRT 1 activators is able to make beneficial, preventive or curative effects for many diseases. And this is precisely related to the interaction between these substances and SIRT 1 pathway.

Among this activators, resveratrol, a known natural substance found in grapes, is an example of a compound that has proved able to stimulate SIRT 1.

Other SIRT 1 stimulants compounds have been described in WO 2013/100111 and in WO 2006/076681.

US20140031299 discloses a combination of resveratrol with a nicotinic acid precursor, US 20110229535 describes combinations of resveratrol with quercetin or piceatannol.

**Description of the invention**

It has now been found that combinations of a first component selected from berberine and tyrosol with a second component selected from quercetin, tyrosol, catechin and ferulic acid, produce synergistic effects on SIRT 1 stimulation significantly greater than those achievable with resveratrol or with the known combinations of resveratrol with other compounds.

Therefore, the invention relates to compositions comprising said combinations.

The compositions of the invention may include for example:

- Berberine associated with at least one compound selected from quercetin, tyrosol, catechin and ferulic acid, or
- Tyrosol associated with at least one compound selected from quercetin, berberine, catechin and ferulic acid.

The compositions of the invention comprise only two of said compounds. Resveratrol is absent. Exemplary compositions include:

- Berberine and tyrosol;

- Tyrosol and ferulic acid;
- Berberine and catechin;
- Tyrosol and catechin.

Particularly preferred compositions are:

- Berberine and tyrosol;

- Tyrosol and ferulic acid.

The compositions of the invention are useful to counteract aging, extend lifespan and for the treatment of pathologies in which SIRT 1 stimulation is desirable, such as type 2 diabetes, acidosis, hyperglycemia, insulin resistance, metabolic syndrome, obesity, liver syndrome and steatosis, fatty liver, hypertriglyceridemia, hypercholesterolemia, hypertension, atherosclerosis, coronary artery disease, peripheral vascular disease, cardiac dysfunction, fibrosis, cardiac and ventricular hypertrophy, cardiac ischemia, hypoxia, asthma, rheumatoid arthritis, Parkinson's disease, Alzheimer's disease, Huntington's Chorea, amyotrophic lateral sclerosis, dementia with Lewy bodies, prion disease, frontotemporal dementia, Wallerian degeneration, macular degeneration, glaucoma, optic neuritis, retinal damage, ischemic proliferative retinopathy, diabetic retinopathy, retinitis pigmentosa, senile cataracts, keratitis, and also in all the carcinogenic degenerations of invasive ductal, invasive lobular, medullary, colloid, papillary and tubular type.

For the envisaged therapeutic uses, the compositions of the invention will be formulated using conventional techniques and excipients. Suitable pharmaceutical forms include tablets, capsules, solutions, suspensions, syrups for oral administration; solutions or emulsions for parenteral administration; gels, creams, ointments, solutions, lotions, powders, emulsions for topical administration. The invention compositions will optionally contain further active ingredients having activities other than that of SIRT 1 stimulation, but such as to usefully complement the activity of the composition. Examples of such additional active ingredients include hypoglycemic, lipid-lowering, anti-hypertensive, anti-inflammatory agents.

The compositions of the invention contain from about 10 mg to about 3000 mg of each of the compounds forming the synergistic combinations here described.

The synergy in SIRT 1 stimulation does not occur for combinations other than those claimed, in particular for combinations of resveratrol, curcumin, niclosamide, malvidin and other. Some of such combinations even proved to induce a decreased expression.

The synergistic effects of co-administration of two substances on SIRT 1 stimulation, determined as a greater, statistically significant effect than the sum of the effects of the two substances individually administered, were evaluated in cell cultures by repeating the experiment at least 8 times for each single combination, either after a stimulation time of 3, 6 and 24 hours (more than 300 total trials), as described in the following detailed description.

**Materials and methods**

**Chemicals and antibodies.**

Cell culture reagents were purchased from Lonza (Basel, Switzerland) and Gibco-BRL (Grand Island, NY). General laboratory chemicals were purchased from Sigma-Aldrich (St. Louis, MO, USA) and Carlo Erba (Milano, Italy). Tyrosol, (+)-Catechin, Malvidin, Berberine Chloride Hydrate, Niclosamide, Curcumin, Quercetin Dihydrate and Resveratrol also were from Sigma-Aldrich (St. Louis, MO, USA).

All substances were dissolved in DMSO (Sigma-Aldrich, St. Louis, MO, USA). The final concentrations of DMSO in culture were between 0.05-0.2%, which had no effect on cell viability.

Ripa Lysis Buffer System, Protease Inhibitor Cocktail, Phosphatase Inhibitor Cocktail C, phenyl-methane-sulfonyl-fluoride (PMSF), Sodium Orthovanadate and Sodium Pyrophosphate were from Santa Cruz Biotechnology (California, USA). CellTiter-Blue Cell Viability Assay was purchased from Promega (Madison, USA).

Reagents, protein markers and membrane for Western Blot were from Biorad Laboratories (California, USA) Rabbit polyclonal antibody against SIRT 1 (H-300, sc-15404), mouse monoclonal antibody against Actin (C-2, sc-8432), goat anti-rabbit IgG-HRP antibody (sc-2004) and goat anti-mouse IgG-HRP antibody (sc-2005) were purchase from Santa Cruz Biotechnology (California, USA). Luminata Crescendo Western HRP Substrate for chemiluminescent detection of bands were from Millipore (Massachusetts, USA).

**Cell culture**

Human Cervical Carcinoma cells (HeLa) were cultured in DMEM supplemented with 10% (v/v) fetal bovine serum, 1% antibiotics (penicillin and streptomycin) and 1% L-glutamine at 37°C in a humidified air atmosphere with 5% (v/v) $CO_2$.

The cells were grown to a confluence of 80%, were then trypsinized, counted with a Burker chamber and plated (100.000 cells /well). The day of the experiment, the cells were treated with the substances at the different concentrations and left in incubation for 3, 6 and 24 hours. The substances were solubilized in DMSO, used at concentrations not toxic for cells (v/v < 0.5%).

At the end of the incubation periods, the cells were washed with PBS (pH 7.4) and collected in a 1.5 ml microtube with 0.130 ml of lysis buffer (RIPA buffer) containing a protease inhibitor cocktail, a phosphatase inhibitor cocktail, PMSF, sodium orthovanadate and sodium pyrophosphate.

They were lysed by three freezing-thawing cycles and then the supernatant were recovered.

A part of the lysate was used to quantified the protein content with the spectrophotometer (Thermo Scientific Multiskan FC, Thermo Scientific), using a commercial kit based on the Bradford assay. The final concentrations are expressed in $\mu g/\mu l$.

**Western Blot Analysis**

The cell lysates (25 $\mu g/\mu l$) were combined with the appropriate amount of 1x SDS sample buffer (0.5 M Tris-HCl pH 6.8, 20% SDS, 10% [vol/vol] glycerol, 5% [vol/vol] $\beta$-mercaptoethanol, 0.2% bromophenol blue), heated at 95°C for 5 min, separated by SDS-PAGE and then transferred to polyvinylidene difluoride membranes (PVDF).

For the electrophoretic separation was used a polyacrylamide gel, composed of two parts, a lower one called "Running gel", containing acrylamide (acrylic acid amide) to 12% (acrylamide/bis-acrylamide 30%), in which occurs the separation of the proteins, and an upper one, called loading gel or "Stacking gel", containing acrylamide to 10%, where were built the wells for sample loading.

The electrophoretic run was performed by passing a current at 200V for about 1 hour, or until when the front has not reached the bottom of the gel.

Once running, the proteins were transferred from the gel to a nitrocellulose membrane (PVDF).

The transfer was performed by passing a voltage of 100V for 1 hour.

The membranes were blocked with 5% (wt/vol) nonfat dry milk in Tris-buffered saline with Tween buffer (T-TBS) (20 mmol/l Tris-Hcl, pH 7.6, 0.138 mol/l NaCl, and 0.1% Tween 20) for 1 hour under stirring and at room temperature.

The expression of SIRT 1 was analyzed incubating the membranes in the presence of specific primary antibodies and, subsequently, of secondary peroxidase-conjugated antibodies, suitable diluted in 5% BSA in T-TBS. Primary Antibodies were used overnight at 4°C at the following conditions: anti-SIRT 1, rabbit (1:1000) and anti-$\beta$-actine, mouse (1:1000). Secondary antibodies were instead used at this dilution: goat anti-rabbit (1:10000) for SIRT 1 and for $\beta$-actin.

During this phase the primary antibody binds to species-specific secondary antibody conjugated to a peroxidase enzyme that will bind, in the next step, a specific substrate (luminol) for the reaction of chemiluminescence.

Later, were performed a series of washes with TBS-Tween under stirring, in order to remove all the unbound secondary antibody.

Bound antibodies were visualized using Chemiluminescence Luminata Crescendo Western HRP Substrate and bands were detected and analyzed by the Kodak Image Station 440CF (Eastman Kodak, Rochester, NY, USA).

Cytotoxicity assay.

CellTiter-Blue Cell Viability Assay (Promega).

The CellTiter-Blue Cell Viability Assay provides a homogeneous, fluorometric method for estimating the number of viable cells present in multiwell plates. It uses the indicator dye resazurin to measure the metabolic capacity of cells an indicator of cell viability. Viable cells retain the ability to reduce resazurin into resorufin, which is highly fluorescent. Nonviable cells rapidly lose metabolic capacity, do not reduce the indicator dye, and thus do not generate a fluorescent signal.

For this purpose, were set up 96-well plates, containing medium, cells to the desired density and the substances to be analyzed, appropriately diluted in DMSO.

More precisely, were seeded 5000 cells per well, in a volume of medium such that the final volume was 100 microliters per well. The cells were treated with the selected substances and incubated for 3, 6,24 and 48 hours.

At the end of treatment, the reagent (20$\mu L$/well) was appropriately added and the plate was incubated in the dark for 4 hours at 37°C. Finally, the fluorescence ($560_{Ex}/590_{Em}$) was detected using the Cytofluor 2300 (Millipore). The fluorescence produced is directly proportional to the number of viable cells and, through the analysis, it was obtained a percentage value compared to the control (cells without treatment).

**Trypan Blue Cytotoxicity Assay.**

The cell suspension (50 uL) was diluted 1: 2 with Trypan blue and a rate of 10 $\mu$l was placed in the chamber of Burker.

Counting the cells present on the slide in a predetermined pattern, and being the volume contained in a single chamber fixed and known, with the correct proportions it can be traced back to the concentration of live cells contained in the starting sample.

Number of living cells = "x" cells translucent (not blue colored) * 10000

(conversion factor) * 2 (dilution factor) * 0:05 (suspension volume expressed in ml).

**Results.**

Table 1 reports the results of the Western blot analysis of the expression of SIRT 1, expressed as a percentage increase compared to control. It can be observed that the expression of this protein is significantly increased in all experimental groups compared to the control group ($p < 0.001***$). In particular, berberine, tyrosol, ferulic acids and catechin induce a greater increase than the other groups, especially with respect to resveratrol, used as a reference standard.

**Table 1**

| Substance and concentration [$\mu$M] | | % 3h increase | % 6h increase | % 24h increase |
|---|---|---|---|---|
| Resveratrol | [5 $\mu$M] | 46 | 47 | 68 |
| Resveratrol | [10 $\mu$M] | 47 | 48 | 69 |
| Berberine | [5 $\mu$M] | 63 | 65 | 75 |
| Berberine | [10 $\mu$M] | 65 | 64 | 75 |
| Quercetin | [5 $\mu$M] | 21 | 22 | 29 |
| Quercetin | [10 $\mu$M] | 27 | 27 | 54 |
| Tyrosol | [10 $\mu$M] | 53 | 62 | 68 |
| Tvrosol | [20 $\mu$M] | 76 | 93 | 101 |
| Catechin | [5 $\mu$M] | 44 | 44 | 46 |
| Catechin | [10 $\mu$M] | 54 | 54 | 57 |
| Ferulic Acid | [25 $\mu$M] | 43 | 44 | 66 |
| Ferulic Acid | [50$\mu$M] | 57 | 58 | 75 |

Table 2 shows the percentage (%) increase of SIRT 1 expression, due to the administration of the two substance [X+Y] in combination, with respect to the sum of the effects of the two substances [X] + [Y] administered separately.

In the analysis of the results a synergistic effect was considered to be present when the simultaneous administration of two substances induces a greater, statistically significant effect on SIRT 1 stimulation, compared to the sum of the effects of the two substances individually administered.

[X+Y] *statistically greater than* [X] + [Y]

where [X + Y], represents the effect obtained by administering simultaneously the two substances X and Y, while [X] + [Y] represents the sum of the effects achieved by administering individually substances X and Y.

So, to verify the presence of a possible synergistic effect between the substances administered individually with respect to those administered in combination on the SIRT 1 stimulation, the compounds were combined two by two, considering for each substance the two concentrations that caused a greater expression of SIRT 1.

In each combinations, the synergistic effect was evaluated after a stimulation time of 3, 6 and 24 hours, and was confirmed by repeating the experiment for a minimum of 8 times for each combination.

The expression of SIRT 1 was significantly increased not only in all experimental groups compared with the control group (p<0.001***), but in all the simultaneous administration groups compared to the individually administered groups (p<0.001 ***, p<0.05 **).

**Table 2**

| Associations and concentrations [µM] | % 3 h increase [X+Y] | % 3h increase [X]+[Y] | Synergic increase [X+Y] respect to [X]+[Y] 3h | % 6h increase [X-Y] | % 6h increase [X]+[Y] | Synergic increase [X+Y] respect to [X]+[Y] 6h | % 24h increase [X+Y] | % 24h increase [X]+[Y] | Synergic increase [X+Y] respect to [X]+[Y] 24h |
|---|---|---|---|---|---|---|---|---|---|
| Berberine [5 µM] Tyrosol [20 µM] | 295 | 139 | 155 $p<0.001$*** | 338 | 158 | 179 $p<0.001$*** | 209 | 173 | 33 $p<0.05$* |
| Berberine [10 µM] Tyrosol [20 µM] | 298 | 141 | 156 $p<0.001$*** | 304 | 157 | 147 $p<0.001$*** | 416 | 176 | 239 $p<0.001$*** |
| Berberine [5 µM] Catechin [10 µM] | 150 | 117 | 33 $p<0.001$*** | 170 | 119 | 50 $p<0.001$*** | 186 | 132 | 54 $p<0.001$*** |
| Berberine [10 µM] Catechin [10 µM] | 168 | 119 | 48 $p<0.001$*** | 186 | 118 | 68 $p<0.001$*** | 198 | 132 | 66 $p<0.001$*** |
| Tyrosol [10 µM] Catechin [10 µM] | 166 | 107 | 58 $p<0.001$*** | 247 | 115 | 131 $p<0.001$*** | 252 | 125 | 127 $p<0.001$*** |
| Tyrosol [20 µM] Catechin [5 µM] | 156 | 120 | 3.6 $p<0.0001$*** | 156 | 137 | 18 $p<0.05$* | 169 | 146 | 22 $p<0.05$* |
| Tyrosol [20 µM] Catechin [10 µM] | 192 | 130 | 61 $p<0.001$*** | 193 | 147 | 46 $p<0.05$* | 196 | 158 | 38 $p<0.05$* |
| Tyrosol [20 µM] Ferulic Acid [20 µM] | 241 | 120 | 121 $p<0.001$*** | 285 | 137 | 148 $p<0.001$*** | 206 | 167 | 38 $p<0.001$*** |
| Tyrosol [20 µM] Ferulic Acid [50 µM] | 267 | 134 | 133 $p<0.001$*** | 304 | 151 | 153 $p<0.001$*** | 304 | 161 | 143 $p<0.001$*** |

EP 3 212 187 B1

**Claims**

1.  Compositions stimulating the expression of Sirtuin 1, selected from combinations of:

    - Berberine and tyrosol;
    - Tyrosol and ferulic acid.

2.  Compositions of claim 1 for use in the treatment and prevention of type 2 diabetes, acidosis, hyperglycemia, insulin resistance, metabolic syndrome, obesity, liver syndrome and steatosis, fatty liver, hypertriglyceridemia, hypercholesterolemia, hypertension, atherosclerosis, coronary artery disease, peripheral vascular disease, cardiac dysfunction, fibrosis, cardiac and ventricular hypertrophy, cardiac ischemia, hypoxia, asthma, rheumatoid arthritis, Parkinson's disease, Alzheimer's disease, Huntington's Chorea, amyotrophic lateral sclerosis, dementia with Lewy bodies, prion disease, frontotemporal dementia, Wallerian degeneration, macular degeneration, glaucoma, optic neuritis, retinal damage, ischemic proliferative retinopathy, diabetic retinopathy, retinitis pigmentosa, senile cataracts, keratitis, carcinogenic degenerations.


**Patentansprüche**

1.  Zusammensetzungen, die die Expression von Sirtuin 1 stimulieren, ausgewählt aus Kombinationen aus:

    - Berberin und Tyrosol;
    - Tyrosol und Ferulinsäure.

2.  Zusammensetzungen nach Anspruch 1 zur Verwendung bei der Behandlung und Vorbeugung von Typ-2-Diabetes, Azidose, Hyperglykämie, Insulinresistenz, metabolischem Syndrom, Adipositas, Lebersyndrom und Steatose, Fettleber, Hypertriglyzeridämie, Hypercholesterinämie, Bluthochdruck, Atherosklerose, koronarer Herzkrankheit, peripherer Gefäßkrankheit, kardialer Dysfunktion, Fibrose, kardialer und ventrikulärer Hypertrophie, kardialer Ischämie, Hypoxie, Asthma, rheumatoide Arthritis, Parkinson-Krankheit, Alzheimer-Krankheit, Chorea Huntington, amyotrophe Lateralsklerose, Lewy-Körperchen-Demenz, Prionen-Krankheit, Frontotemporale Demenz, Wallerische Degeneration, Makuladegeneration, Glaukom, Sehnervenentzündung, Netzhautschäden, ischämische proliferative Retinopathie, diabetische Retinopathie, Retinitis pigmentosa, Grauer Star, Keratitis, karzinogene Degenerationen.


**Revendications**

1.  Compositions stimulant l'expression de la sirtuine 1, choisies parmi des combinaisons de :

    - Berbérine et tyrosol ;
    - Tyrosol et acide férulique.

2.  Compositions selon la revendication 1 pour une utilisation dans le traitement et la prévention du diabète de type 2, de l'acidose, de l'hyperglycémie, de la résistance à l'insuline, du syndrome métabolique, de l'obésité, du syndrome et de la stéatose hépatiques, du foie gras, de l'hypertriglycéridémie, de l'hypercholestérolémie, de l'hypertension, de l'athérosclérose, de la maladie des artères coronaires, de la maladie vasculaire périphérique, du dysfonctionnement cardiaque, de la fibrose, de l'hypertrophie cardiaque et ventriculaire, de l'ischémie cardiaque, de l'hypoxie, de l'asthme, de la polyarthrite rhumatoïde, de la maladie de Parkinson, de la maladie d'Alzheimer, de la chorée de Huntington, de la sclérose latérale amyotrophique, de la démence à corps de Lewy, d'une maladie à prions, de la démence frontotemporale, de la dégénérescence wallerienne, de la dégénérescence maculaire, du glaucome, de la névrite optique, de lésions rétiniennes, de la rétinopathie ischémique proliférative, de la rétinopathie diabétique, de la rétinite pigmentaire, des cataractes séniles, de la kératite, de la dégénérescence cancérigène.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013100111 A
- WO 2006076681 A
- US 20140031299 A
- US 20110229535 A